# EUROPÄISCHE PATENTSCHRIFT

(11) **EP 0 910 510 B1**
(45) Veröffentlichungstag und Bekanntmachung des Hinweises auf die Patenterteilung: **23.08.2000**
(21) Anmeldenummer: 97925985.0
(22) Anmeldetag: 06.06.1997
(51) Int. Cl.: B43K 5/18, A61M 25/00

(54) **VENTILANORDNUNG ZUR STEUERUNG EINES FLUIDDURCHFLUSSES ZWISCHEN ZWEI FLUIDRÄUMEN UND DAMIT VERSEHENES SCHREIBGERÄT**
VALVE ARRANGEMENT FOR CONTROLLING A FLOW OF FLUID BETWEEN TWO FLUID CHAMBERS AND WRITING IMPLEMENT PROVIDED THEREWITH
CONFIGURATION DE CLAPETS POUR ASSURER LA COMMANDE DU DEBIT FLUIDIQUE ENTRE DEUX CHAMBRES FLUIDIQUES ET INSTRUMENT A ECRIRE MUNI D'UNE TELLE CONFIGURATION DE CLAPETS

(30) Priorität: 03.07.1996 DE 19626755
(43) Veröffentlichungstag der Anmeldung: 28.04.1999
(73) Patentinhaber: Weiss, Oliver, 90530 Wendelstein (DE)
(72) Erfinder: Weiss, Oliver, 90530 Wendelstein (DE)
(74) Vertreter: Hübner, Gerd, Dipl.-Phys.
(86) Internationale Anmeldenummer: EP9702935
(87) Internationale Veröffentlichungsnummer: WO9801307

(56) Entgegenhaltungen:
- EP-A- 0 125 844
- EP-A- 0 250 891
- EP-A- 0 476 796
- WO-A-95/11283
- FR-A- 1 360 234

## Beschreibung

Die Erfindung betrifft eine Ventilanordnung zur Steuerung eines Fluiddurchflusses zwischen einem ersten und einem zweiten Fluidraum sowie ein damit ausgerüstetes Schreibgerät.

Grundsätzlich besteht derzeit aufgrund der in vielen technischen Gebieten stattfindenden Miniaturisierung der Bedarf nach konstruktiv einfach aufgebauten, miniaturisierbaren Ventilen, die u. a. zu Steuerungszwecken in der Mikropneumatik und Mikrohydraulik eingesetzt werden können. Auch die Medizin- und Schreibgerätetechnik [siehe z.B. WO-A- 9511283] verlangt nach solchen Ventilen.

Aus der EP-A-250 891 ist eine Ventilanordnung im Bereich einer Katheterkonstruktion gemäß dem Oberbegriff des Patentanspruches 1 bekannt. Dabei ist ein langgestrecktes, an einem Ende geschlossenes Schlauchstück vorgesehen, das bis auf einen Ringbereich am Ende durch eine Einlage versteift ist. An einem Handknaufist ein Drehknopf gelagert, der mit einer stilettartigen Betätigungsstange im Lumen des Schlauchstückes verbunden ist, die wiederum zu dessen freien Ende verläuft und dort verankert ist. Durch Drehung des Knaufes ist der Kopf des Schlauchstückes über die Betätigungsstange relativ zum restlichen Schlauchstück verdrehbar, so daß der unverstärkte Bereich in sich tordiert wird. Dort vorgesehene Schlitze öffnen sich durch diese Torsion und geben den Flüssigkeitsdurchgang zwischen dem Lumen des Schlauchstückes und dem Außenraum frei.

Nachteilig bei der bekannten Anordnung ist die Tatsache, daß für die Erzeugung der Torsion des unverstärkten Abschnittes des Schlauchstückes eine relativ aufwendige Vorrichtung gegeben sein muß. Insbesondere müssen zwei Bereiche des Schlauchstückes, nämlich einerseits der verstärkte Bereich und andererseits die Kappe in sich steif, jedoch über ein flexibles Zwischenstück miteinander verbunden sein, um eine Torsion dieses Zwischenstücks zur Ventilbetätigung zu ermöglichen. Ferner ist die Drehbetätigung des Betätigungsteiles zur Erzeugung der Torsion nur mit einer aufwendigen Knauf-Knopf-Handhabe erzielbar.

Beim Stand der Technik besteht also das Problem, daß eine relativ aufwendig konstruierte Ventilanordnung auch nur sehr umständlich betätigbar ist.

Zur Lösung dieser Problematik schlägt die Erfindung eine Ventilanordnung der eingangs genannten Art mit folgenden Merkmalen vor:
- ein Schlauchstück aus Elastomermaterial, dessen Wandaußenseite mit einem ersten Fluidraum in Kontakt steht und dessen Innenraum oder Lumen mit einem zweiten Fluidraum in Verbindung steht,
- ein oder mehrere durchgehende Schlitze in der Wand des Schlauchstükkes, die in dessen undeformierten Zustand aufgrund der materialinhärenten Rückstellkraft des Elastomermaterials geschlossen und durch eine Deformation des Schlauchstückes zu öffnen sind, und
- ein Betätigungsteil am Schlauchstück, mittels dessen radialer Auslenkung über eine darauf aufgebrachte Kraft das Schlauchstück zur Steuerung des Fluiddurchflusses durch die Schlitze quer zum Schlauchstück deformierbar ist.

Aus der vorstehenden Merkmalskombination wird deutlich, daß es sich bei dem Erfindungsgegenstand um ein mechanisch zu betätigendes Ventil für die Durchlaßsteuerung von Gasen und Flüssigkeiten handelt. Die beiden erwähnten Fluidräume können dabei zwei abgeschlossene Volumina oder auch einen Vorratsraum und die Umgebung oder Atmosphäre sein, wie es im folgenden noch näher beispielhaft erläutert wird.

Das Schlauchstück besteht vorteilhafterweise aus Silikonmaterial. Dies vermindert den Fertigungsaufwand für die erfindungsgemäße Ventilanordnung, da Silikonschläuche bereits in vielen Abmessungen und Qualitäten als handelsübliche Massenartikel erhältlich sind. Ferner eröffnet sich dadurch besonders der medizinische Bereich als Anwendungsfall für das Ventil, da Hautverträglichkeit und Körperakzeptanz bei Silikonmaterial gegeben sind.

Bevorzugterweise ist das Betätigungsteil als im Lumen des Schlauchstückes sitzendes, vor dessen Schlitzbereich endendes Rohrstück ausgebildet. Diese Konstruktion gewährleistet geringe Betätigungskräfte und eine einwandfreie Ableitung des durch die deformationsbedingt geöffneten Schlitze durchtretenden Fluids vom ersten zum zweiten Fluidraum.

Die Anordnung und die genaue Ausbildung der Schlitze im Schlauchstück sind jeweils anwendungsorientiert festzulegen. Es muß lediglich gewährleistet sein, daß im undeformierten Zustand des Schlauchstückes die Schlitze geschlossen sind. Es muß sich also dabei um feine Schnitte und nicht grobe Aussparungen in der Schlauchwandung handeln.

Um eine von der Richtung der Deformation unabhängige Ansprechcharakteristik des Ventils zu erzielen, ist zu bevorzugen, eine oder mehrere peripher umlaufende Schlitzreihen im Schlauchstück vorzusehen.

Durch eine fluiddichte Lagerung des Schlauchsstückes jeweils vor seinen beiden Enden wird eine saubere Fixierung des Schlauchstückes und Abtrennung zwischen den beiden Fluidräumen erzielt.

Zusammenfassend sind als Vorteile zu der erfindungsgemäßen Ventilanordnung noch deren Korrisionsbeständigkeit und sehr hohe chemische Beständigkeit insbesondere gegen Säuren und Laugen - es brauchen keine metallischen Teile verwendet zu werden -, der sehr geringe Platzbedarf und die geringen Betätigungskräfte zu nennen. Ferner ist die Ventilanordnung in einem großen Temperaturbereich einsetzbar. Nicht zuletzt werden nur Materialien verwendet, die eine Funkenbildung ausschließen, so daß die Ventilanordnung auch in explosionsgefährdeter Umgebung einsetzbar ist.

Als bevorzugte Anwendung der erfindungsgemäßen Ventilanordnung ist der Einsatz in einem Schreibgerät zur Regulierung des Tintenflusses zwischen einem Tintenreservoir des Schreibgerätes und dessen Schreibspitze vorgesehen.

Hinsichtlich der beanspruchten Weiterbildungen des Schreibgerätes mit einer solchen Ventilanordnung wird zur Vermeidung unnötiger Wiederholungen auf die folgende Beschreibung verwiesen, in der die Ventilanordnung selbst und ein damit versehenes Schreibgerät mit weiteren Merkmalen, Einzelheiten und Vorteilen anhand der beigefügten Zeichnungen näher erläutert wird. Es zeigen:
- Fig. 1 und 2: Schematische Ansichten der Ventilanordnung in geschlossenem und geöffneten Zustand,
- Fig. 3 und 4: eine vergrößerte Darstellung des Schlauchstückes in unbeanspruchtem und deformierten Zustand, und
- Fig. 5 und 6: schematische Längsschnitte durch einen Markierstift bei geschlossener und geöffneter Ventilanordnung.

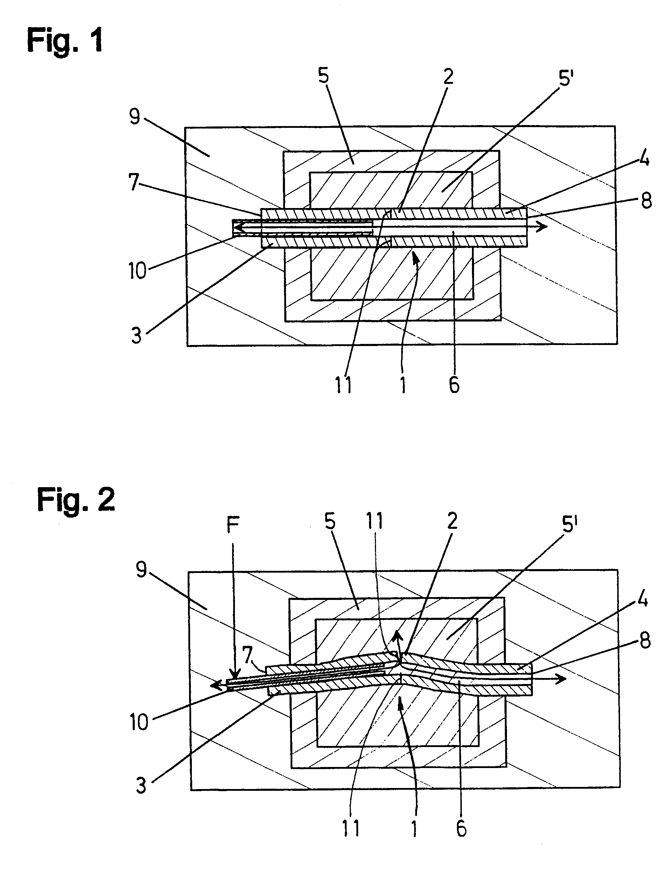

In den Fig. 1 und 2 ist höchst schematisch die erfindungsgemäße Ventilanordnung 1 dargestellt. Deren Herzstück ist ein kurzes Schlauchstück 2 aus Silikonmaterial, das vor seinen beiden offenen Enden 3, 4 fluiddicht in einem Gehäuseteil 5 gelagert ist. Das Gehäuseteil 5 gehört zu einem ersten Fluidraum 5', in dem ein Fluid, z. B. eine Flüssigkeit steht. Das Schlauchstück 2 ist also zwischen den Lagerstellen an den beiden offenen Enden an seiner Wandaußenseite von der Flüssigkeit im ersten Fluidraum 5' umgeben. Das Lumen 6 des Schlauchstückes 2 mündet mit seinen beiden Öffnungen 7, 8 in einen zweiten Fluidraum, der in Fig. 1 und 2 als Ganzes mit 9 bezeichnet und durch eine breitschraffierte Fläche angedeutet ist. In das Lumen 6 des Schlauchstückes 2 ist durch die eine Öffnung 7 hindurch ein Rohrstück 10 als Betätigungsteil eingeschoben, das kurz vor dem Bereich der Schlitze 11 in dem Schlauchstück 2 endet.

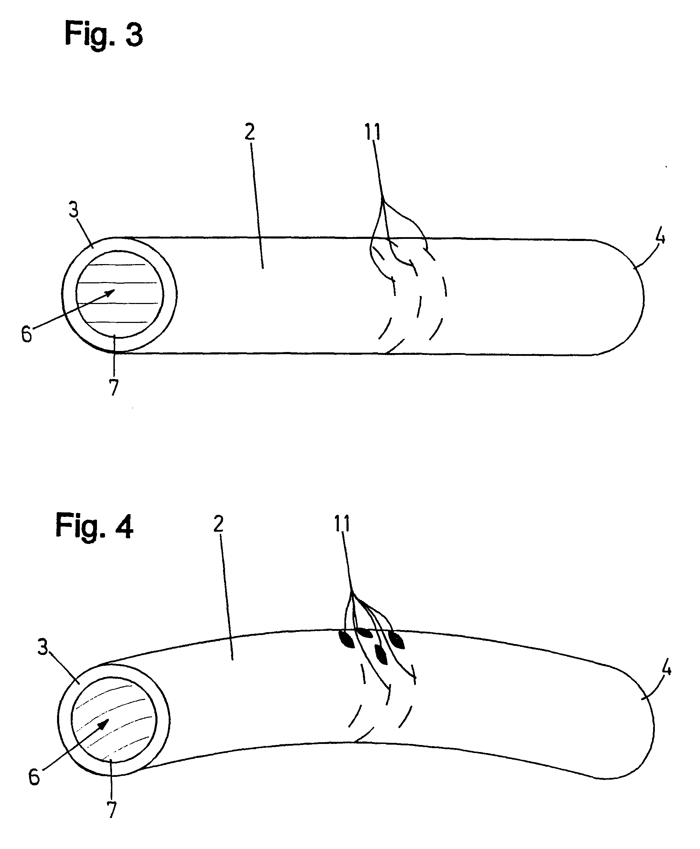

Ein Beispiel für die Formgebung und Ausgestaltung der Schlitze 11 ist in den Fig. 3 und 4 dargestellt. Es sind drei Reihen peripher umlaufender Schlitze 11 etwa mittig bezogen auf die Länge des Schlauchstückes 2 vorgesehen. Bei den Schlitzen 11 handelt es sich um mikrofeine Schnitte, die durch die Schlauchwand verlaufen.

In Fig. 1 ist die Ventilanordnung 1 im geschlossenen Zustand dargestellt. Durch die dem Schlauchstück 2 innewohnende, materialinhärente Rückstellkraft seines Silikon-Elastomermaterials sind die Schlitze 11 geschlossen, so daß auch eine mit leichtem Überdruck im Gehäuseteil 5 (erster Fluidraum 5') anstehende Flüssigkeit nicht durch die Schlitze 11 in das Lumen 6 des Schlauchstückes 2 eintreten kann.

Bei Einwirkung einer Kraft F auf das aus dem Schlauchstück 2 hinausragende Rohrstück 10 wird das Schlauchstück 2 ausgelenkt und deformiert, wie in Fig. 2 erkennbar ist. Die Schlitze 11, die auf der gedehnten Seite des Schlauchstückes 2 liegen, werden durch diese Deformation geöffnet und es kann Flüssigkeit oder Gas von dem Gehäuseteil 5 (erster Fluidraum 5') durch die Schlitze in das Lumen 6 des Schlauchstückes 2 und von dort über die Öffnungen 7, 8 in den zweiten Fluidraum 9 übertreten. Die erwähnte Öffnung der Schlitze 11 ist in Fig. 4 übertrieben, aber augenfällig dargestellt.

Wirkt die Kraft F nicht mehr ein, so stellt sich das Schlauchstück 2 wieder gerade und die Schlitze 11 werden rundum geschlossen. Die Fluidverbindung zwischen dem ersten Fluidraum 5' und dem zweiten Fluidraum 9 ist damit wieder unterbrochen.

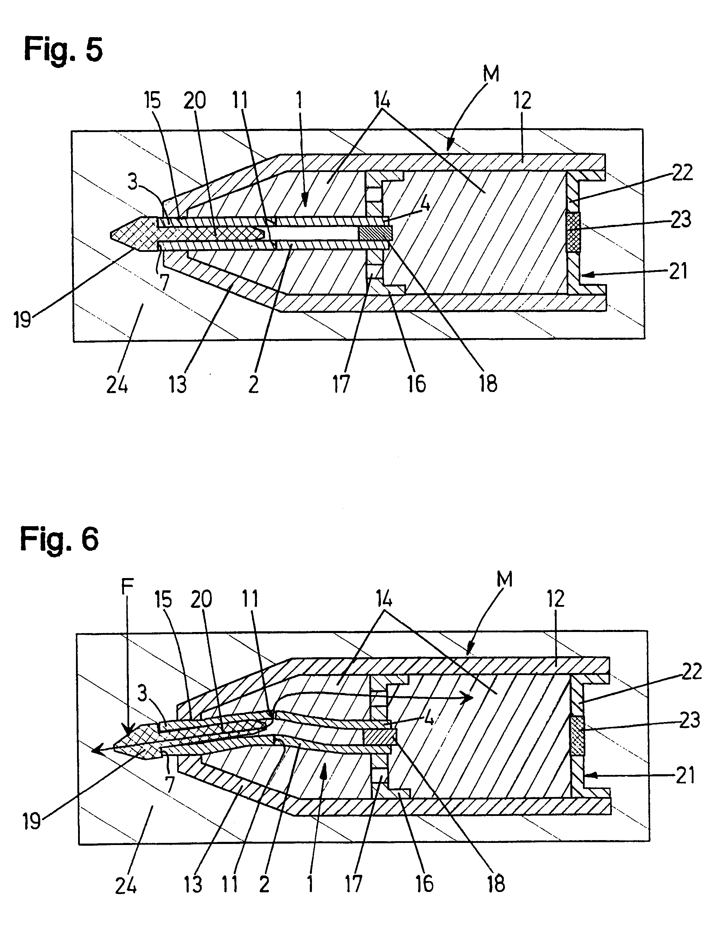

In den Fig. 5 und 6 ist ein praktisches Beispiel für die Anwendung der in den Fig. 1 bis 4 dargestellten Ventilanordnung gezeigt. Es handelt sich dabei um einen schematisch im Schnitt dargestellten Markierstift M, der ein übliches zylindrisches oder abgeplattet rohrförmiges Gerätegehäuse 12 mit konisch zulaufender Gehäusespitze 13 aufweist. Das Gerätegehäuse 12 umschließt ein Reservoir 14 für die Schreibflüssigkeit des Markierstiftes

In der Gehäusespitze 13 ist eine Öffnung 15 vorgesehen, in der das eine Ende 7 des Schlauchstückes 2 mit seiner Außenseite fluiddicht abschließend gelagert ist. Das koaxial zur Längsachse des Gerätegehäuses 12 angeordnete Schlauchstück 2 ist ferner mit seinem anderen Ende 4 in einem Halteelement 16 im Gerätegehäuse 12 festgelegt, das Durchtrittsöffnungen 17 für die Schreibflüssigkeit aufweist. Die am Ende 4 liegende Öffnung 8 des Schlauchstückes 2 ist durch einen Pfropfen 18 in Form eines Silikonschnurstückes dicht verschlossen. In die gegenüberliegende Öffnung 7, die außerhalb des Gerätegehäuses 12 liegt, ist die Schreibspitze 19 des Markierstiftes M mit ihrem Schaft 20 eingesetzt. Die Schreibspitze 19 selbst bildet das Betätigungsteil für die Ventilanordnung 1, wobei ihr Schaft 20 wiederum bis kurz vor die Schlitze 11 im Schlauchstück 2 reicht. Die Schreibspitze 19 mit Schaft 20 ist ferner aus Fasermaterial gefertigt, wie dies bei Markierstiften M zum Transport der Schreibflüssigkeit aufgrund Kapillareinwirkung üblich ist.

Die der Gehäusespitze 13 abgewandte Öffnung 21 des Gerätegehäuses ist im übrigen durch eine Kappe 22 verschlossen, in der ein Druckausgleichselement 23 in Form einer hydrophoben, semipermeablen Membran eingesetzt ist. Letztere ist mit der Kappe 22 ultraschall verschweißt.

Anhand der Fig. 6 ist die Steuerung des Tintenflusses bei dem Markierstift M zu erläutern:

Wird der Markierstift M auf ein Papierblatt 24 aufgesetzt, wie dies im Fig. 6 höchst schematisch angedeutet ist, und dabei die Kraft F auf die Schreibspitze 19 aufgebracht, so wird das Schlauchstück 2 wiederum vom Schaft 20 der Schreibspitze 19 ausgelenkt und deformiert. Dadurch öffnen sich die Schlitze 11 in dem Schlauchstück 2, so daß die im Reservoir 14 (erster Fluidraum) vorhandene Flüssigkeit in das Lumen 6 des Schlauchstückes 2 eintreten kann und über die Kapillarkräfte in der Schreibspitze 19 zum Papierblatt 24 transportiert wird. Letzteres bildet entsprechend der vorstehenden Terminologie praktisch den zweiten Fluidraum. Sobald der Markierstift M von der Unterlage genommen wird und die Kraft F entfällt, stellt sich das Schlauchstück 2 wieder gerade und der Tintenfluß vom Reservoir 14 zur Schreibspitze 19 ist unterbrochen.

Abschließend werden die Vorteile des erfindungsgemäßen Schreibgerätes, wie es vorstehend beschrieben wurde, zusammengefaßt:
- Die Regulierung des Tintenflusses erfolgt durch ein unkompliziertes, kostengünstiges Ventilsystem.
- Das Funktionsprinzip ist für vielerlei Arten von Schreibpasten und - flüssigkeiten wie auch für Kosmetikflüssigkeiten verwendbar.
- Der Tankinhalt des Schreibgerätes wird nur durch die Gehäusegröße bestimmt und ist damit innerhalb bestimmter Grenzen beliebig.
- Mehr als 90 % der Schreibflüssigkeit sind nutzbar. Daraus resultiert eine wesentlich höhere Ergiebigkeit bei gleicher Füllmenge im Vergleich zu Faserspeichern.
- Das Schreibverhalten ist unabhängig vom Rest-Tankinhalt.
- Das Schreibgerät ist schocksicher, d. h. beim Hinunterfallen erfolgt kein plötzliches Austreten von Schreibflüssigkeit und damit verbundenes Klecksen.
- Das Schreibgerät kann nachfüllbar ausgestaltet werden.
- Die Schreibspitze kann beliebig geformt und austauschbar ausgelegt werden, da sie nur in das Schlauchstück eingesteckt ist.
- Das Schreibgerät ist optimal recyclingfähig, da die Einzelteile aufgrund der einfachen Konstruktion leicht zu demontieren und separieren sind.

## Patentansprüche

1. Ventilanordnung zur Steuerung eines Fluiddurchflusses zwischen einem ersten und einem zweiten Fluidraum mit
- einem Schlauchstück (2) aus Elastomermaterial, dessen Wandaußenseite mit dem ersten Fluidraum (5', 14) in Kontakt steht und dessen Lumen (6) mit dem zweiten Fluidraum (9, 24) in Verbindung steht,
- einem oder mehreren durchgehenden Schlitzen (11) in der Wand das Schlauchstückes (2), die in undeformiertem Zustand des Schlauchstükkes (2) aufgrund der materialinhärenten Rückstellkraft des Elastomermaterials geschlossen und durch eine Deformation des Schlauchstückes (2) zu öffnen sind, und
- einem Betätigungsteil (10, 19) am Schlauchstück (2), **dadurch gekennzeichnet, daß** das Schlauchstück (2) mittels einer durch radiale Auslenkung über das Betätigungsteil (10, 19) aufgebrachten Kraft (F) zur Steuerung des Fluiddurchflusses durch die Schlitze (11) quer zum Schlauchstück (2) deformierbar ist.

2. Ventilanordnung nach Anspruch 1, **dadurch gekennzeichnet, daß** das Schlauchstück (2) aus Silikonmaterial besteht.

3. Ventilanordnung nach Anspruch 1 oder 2, **dadurch gekennzeichnet, daß** das Betätigungsteil als im Lumen (6) des Schlauchstückes (2) sitzendes, vor dessen Schlitzen (11) endendes Rohrstück (10) ausgebildet ist.

4. Ventilanordnung nach einem der Ansprüche 1 bis 3, **dadurch gekennzeichnet, daß** eine oder mehrere peripher umlaufende Reihen von Schlitzen (11) im Schlauchstück (2) vorgesehen sind.

5. Ventilanordnung nach einem der Ansprüche 1 bis 4, **dadurch gekennzeichnet, daß** das Schlauchstück (2) jeweils vor seinen beiden Enden (3, 4) fluiddicht gelagert ist.

6. Schreibgerät mit einer Ventilanordnung zur Regulierung des Tintenflusses zwischen einem Tintenreservoir (14) und einer Schreibspitze (19), wobei die Ventilanordnung versehen ist mit einem Betätigungsteil (10, 19) am Schlauchstück (2), mittels dessen über eine aufgebrachte Kraft (F) das Schlauchstück (2) zur Steuerung des Fluiddurchflusses durch die Schlitze (11) quer zum Schlauchstück (2) deformierbar ist.

7. Schreibgerät nach Anspruch 6, **dadurch gekennzeichnet, daß** das Schlauchstück (2) der Ventilanordnung (1) im Gerätegehäuse (12) derart gelagert ist, daß ein Ende (3) des Schlauchstückes (2) aus dem Gerätegehäuse (12) nach außen mündet und seine Mündungsöffnung (7) mit der Schreibspitze (19) versehen ist, daß das zweite Ende (4) des Schlauchstückes (2) verschlossen ist, und daß der innerhalb des Gerätegehäuses (12) liegende, mit dem einen oder mehreren Schlitzen (11) versehene Teil des Schlauchstückes (2) auf seiner Außenseite mit dem Tintenreservoir (14) des Schreibgerätes in Verbindung steht.

8. Schreibgerät nach Anspruch 7, **dadurch gekennzeichnet, daß** die Schreibspitze (19) mit einem in das Lumen (6) des Schlauchstückes (2) hineinragenden Schaft (20) versehen ist.

9. Schreibgerät nach Anspruch 8, **dadurch gekennzeichnet, daß** der Schaft (20) in unmittelbarer Nähe des mindestens einen Schlitzes (11) der Ventilanordnung (1) endet.

10. Schreibgerät nach einem der Ansprüche 6 bis 9, **dadurch gekennzeichnet, daß** das der Schreibspitze (19) abgewandte Ende des Schlauchstükkes (2) in einem Halteelement (16) innerhalb des Gerätegehäuses (12) fixiert ist.

## Claims

1. Valve arrangement for control of a fluid through-flow between a first and a second fluid chamber with
- a hose part (2) of elastomer material, the outer wall side of which is in contact with the first fluid chamber (5', 14) and the lumen (6) of which is joined with the second fluid chamber (9, 24);
- one or more through slots (11) in the wall of the hose part (2), which is (are) closed when the hose part (2) is in an undeformed state due to the restoring force inherent in the elastomer material, and which can be opened due to a deformation of the hose part (2); and
- an actuation part (10, 19) on the hose part (2), **characterized in that** said hose part (2) can be deformed transversely to itself by means of a force (F) applied by radial deflection via the actuation part (10, 19) for control of fluid flow through the slots (11).

2. Valve arrangement according to claim 1, **characterized in that** the hose part (2) is comprised of silicone material.

3. Valve arrangement according to claim 1 or 2, **characterized in that** the actuation part is formed as a tube piece (10) sitting in the lumen (6) of the hose part (2) and ending in front of its slots (11).

4. Valve arrangement according to one of claims 1 to 3, **characterized in that** one or more peripherally running rows of slots (11) are provided in the hose part (2).

5. Valve arrangement according to one of claims 1 to 4, **characterized in that** the hose part (2) is mounted in a fluid-tight manner in front of each of its two ends (3, 4).

6. Writing utensil with a valve arrangement for regulating the flow of ink between an ink reservoir (14) and a writing tip (19), the valve arrangement being provided with an actuation part (10, 19) on the hose part (2), by means of which, via an applied force (F), said hose part (2) is deformable transversely to the hose part (2) for control of fluid flow through the slots (11).

7. Writing utensil according to claim 6, **characterized in that** the hose part (2) of the valve arrangement (1) is mounted in the utensil housing (12) in such a way that one end (3) of the hose part (2) opens outwardly from the utensil housing (12) and its mouth opening (7) is provided with the writing tip (19) such that the second end (4) of the hose part (2) is closed and that the part of the hose part (2) provided with the one or more slots (11) and lying inside the utensil housing (12) connects on its outer side with the ink reservoir (14) of the writing utensil.

8. Writing utensil according to claim 7, **characterized in that** the writing tip (19) is provided with a shaft (20) protruding into the lumen (6) of the hose part (2).

9. Writing utensil according to claim 8, **characterized in that** the shaft (20) terminates in the direct vicinity of the at least one slot (11) of the valve arrangement (1).

10. Writing utensil according to one of claims 6 to 9, **characterized in that** the end of the hose part (2) turned away from the writing tip (19) is attached in a mounting element (16) inside the utensil housing (12).

## Revendications

1. Dispositif formant clapet pour la commande d'un débit fluidique entre une première chambre fluidique et une deuxième chambre fluidique, comportant
- un morceau de tuyau (2) en élastomère dont le côté extérieur de paroi est en contact avec la première chambre fluidique (5', 14) et dont le lumen (6) est en liaison avec la deuxième chambre fluidique (9, 24),
- une ou plusieurs fentes traversantes (11) dans la paroi du morceau de tuyau (2) qui sont fermées, lorsque le morceau de tuyau (2) n'est pas déformé, en raison de la force de rappel, inhérente au matériau, de l'élastomère et qui sont à ouvrir par une déformation du morceau de tuyau (2),
- une partie d'actionnement (10, 19) sur le morceau de tuyau (2),
caractérisé en ce que le morceau de tuyau (2) est déformable au moyen d'une force (F), appliquée par une déviation radiale par l'intermédiaire de la partie d'actionnement (10, 19), pour commander le débit fluidique à travers les fentes (11) transversalement par rapport au morceau de tuyau (2).

2. Dispositif formant clapet selon la revendication 1, caractérisé en ce que le morceau de tuyau (2) est en silicone.

3. Dispositif formant clapet selon la revendication 1 ou 2, caractérisé en ce que la partie d'actionnement est conçue comme un morceau de tube (10) placé dans le lumen (6) du morceau de tuyau (2) et se terminant avant les fentes (11) de celui-ci.

4. Dispositif formant clapet selon l'une des revendications 1 à 3, caractérisé en ce qu'une ou plusieurs rangées, faisant le tour comme une périphérie, de fentes(11) sont prévues dans le morceau de tuyau (2).

5. Dispositif formant clapet selon l'une des revendications 1 à 4, caractérisé en ce que le morceau de tuyau (2) est monté à chaque fois étanche au fluide avant ses deux extrémités (3, 4).

6. Instrument pour écrire avec un dispositif formant clapet pour la régulation du flux d'encre entre un réservoir d'encre (14) et une pointe d'écriture (19), le dispositif formant clapet étant muni d'une partie d'actionnement (10, 19) sur le morceau de tuyau (2), partie d'actionnement au moyen de laquelle le morceau de tuyau (2) est déformable par l'intermédiaire d'une force appliquée (F) pour commander le débit de fluide à travers les fentes (11) transversalement par rapport au morceau de tuyau (2).

7. Instrument pour écrire selon la revendication 6, caractérisé en ce que le morceau de tuyau (2) du dispositif clapet (1) est monté dans le corps d'instrument (12) de telle sorte qu'une extrémité (3) du morceau de tuyau (2) débouche du corps d'instrument (12) vers l'extérieur et son ouverture (7) est munie de la pointe d'écriture (19), que la deuxième extrémité (4) du morceau de tuyau (2) est fermée et que la partie, située à l'intérieur du corps d'instrument (12) et munie d'une ou plusieurs fentes (11), du morceau de tuyau (2) est en liaison par son côté extérieur avec le réservoir d'encre (14) de l'instrument pour écrire.

8. Instrument pour écrire selon la revendication 7, caractérisé en ce que la pointe d'écriture (19) est munie d'une tige (20) pénétrant dans le lumen (6) du morceau de tuyau (2).

9. Instrument pour écrire selon la revendication 8, caractérisé en ce que la tige (20) se termine à proximité immédiate de la ou des fentes (11) du dispositif formant clapet (1).

10. Instrument pour écrire selon l'une des revendications 6 à 9, caractérisé en ce que l'extrémité, opposée à la pointe d'écriture (19), du morceau de tuyau (2) est fixée dans un élément de maintien (16) à l'intérieur du corps d'instrument (12).
